# EUROPEAN PATENT SPECIFICATION

(11) **EP 4 598 417 B1**
(45) Date of publication and mention of the grant of the patent: **29.07.2026**
(21) Application number: 23782166.5
(22) Date of filing: 26.09.2023
(51) Int. Cl.: A61B 5/00, A61B 1/05

(54) **SPACER FOR SENSOR IN INTRALUMINAL SENSING DEVICE**
ABSTANDSHALTER FÜR SENSOR IN INTRALUMINALER MESSVORRICHTUNG
ESPACEUR POUR CAPTEUR DANS UN DISPOSITIF DE DÉTECTION INTRALUMINAL

(30) Priority: 07.10.2022 US 202263414017 P
(43) Date of publication of application: 13.08.2025
(73) Proprietor: Koninklijke Philips N.V., 5656 AE Eindhoven (NL)
(72) Inventor: PARKER, Steven Patrick, 5656 AG Eindhoven (NL)
(74) Representative: Philips Intellectual Property & Standards
(86) International application number: PCT/EP2023/076457
(87) International publication number: WO 2024/074339

(56) References cited:
- US-A1- 2017 064 162
- US-A1- 2021 267 709

## Description

### TECHNICAL FIELD

The subject matter described herein relates to a spacer that fits within a sensor housing for physiology sensing intraluminal devices. This sensor spacer has particular but not exclusive utility for intravascular catheters and guidewires.

### BACKGROUND

Coronary artery disease (CAD) is among the world's leading causes of death. To address this problem, image guided therapy (IGT) makes use of a wide variety of imaging modalities (e.g., coronary angiography) as well as in-body diagnostic devices (e.g. pressure-sensing guidewires or intravascular ultrasound catheters). Small-diameter medical devices such as intraluminal (e.g., intravascular) catheters and guidewires may incorporate sensors (e.g., pressure, temperature, flow, or imaging sensors) whose power and communications occur through electrical conductor bundles. Such devices are known from US 2017/064162 A1 and US 2021/267709 A1**.** However, recent guidewire devices may in some cases have diameters of 360 microns or smaller. Current construction of such devices may require microcables/filars to be hand soldered/bonded to the sensor. Additionally, the subassembly consisting of the sensor and filars may be quite delicate and, if installed incorrectly, may be at risk of electrical short against the conductive sensor housing. Even under high magnification, manual assembly of these assemblies may be extremely challenging, resulting in high scrap rates.

The information included in this Background section of the specification, including any references cited herein and any description or discussion thereof, is included for technical reference purposes only and is not to be regarded as subject matter by which the scope of the disclosure is to be bound.

### SUMMARY

A blood flow velocity sensing guidewire can be used for example to assess Non-Obstructive Coronary Artery Disease (NOCAD) and MicroVascular Disease (MVD). The present disclosure provides a spacer that fits within the sensor housing, between the sensor and a tapered region of the sensor housing interior. For example, micro-scale 3D printing can be used to create a spacer to be placed between the proximal face of the sensor and the distal face of the sensor housing to control the position and orientation of the sensor relative to the sensor housing. The sensor spacer (1) limits the depth that the sensor can go within the sensor housing; (2) helps align the sensor such that the distal surface of the sensor is orthogonal to the longitudinal axis of the sensor housing; (3) centers the sensor within the sensor housing; and (4) prevents the proximal end of the sensor from electrically shorting against the conductive material of the sensor housing. The sensor spacer thus facilitates a more robust assembly process with less risk of scrap. The sensor spacer described herein has particular, but not exclusive, utility for intraluminal medical catheters and guidewires (e.g., intravascular catheter and catheters).

One general aspect includes an intraluminal device. The intraluminal device includes a flexible elongate member configured to extend in a longitudinal direction within a body lumen of a patient; a sensor disposed at a distal region of the flexible elongate member, where the sensor is configured obtain intraluminal data associated with the body lumen; a housing at least partially surrounding the sensor; and a spacer disposed between a portion of the sensor housing and a proximal face of the sensor. The spacer includes: a base; a through-hole extending through the base; a recess disposed distal of the base and surrounded at least partially by a side wall extending distally from the base, where the side wall is configured not to contact a proximal surface of the sensor; a plurality of support ledges projecting radially inward from the side wall and configured to contact the proximal surface of the sensor; and at least one retention feature extending distally from at least one support ledge of the plurality of support ledges and configured to contact a side surface of the sensor.

Implementations may include one or more of the following features. In some embodiments, the spacer is configured to center the sensor within the sensor housing relative to a longitudinal axis of the sensor housing. In some embodiments, the spacer is configured to control a depth of the sensor within the sensor housing along a longitudinal axis of the sensor housing. In some embodiments, the spacer is configured to control an alignment of the sensor relative to a longitudinal axis of the sensor housing. In some embodiments, the spacer includes a first polymer material. In some embodiments, the sensor is adhered to the spacer by a second polymer material positioned at least partially within the recess. In some embodiments, the spacer is adhered to the sensor housing by a third polymer material in contact with a proximal surface of the spacer. In some embodiments, the intraluminal device further including at least two electrical wires extending distally through the through-hole and fixedly attached to and electrically coupled with the sensor. In some embodiments, the at least one retention feature includes a plurality of tabs, where each tab of the plurality of tabs extends distally from a support ledge of the plurality of support ledges. In some embodiments, the retention feature includes the side wall or a counterbore thereof.

One general aspect includes a method for assembling an intraluminal device. The method includes obtaining a flexible elongate member configured to extend in a longitudinal direction within a body lumen of a patient; positioning a sensor housing at a distal region of the flexible elongate member; positioning a spacer within the sensor housing; positioning a sensor at least partially within the sensor housing distal of the spacer, where the sensor is configured obtain intraluminal data associated with the body lumen, where the spacer includes: a base; a through-hole extending through the base; a recess disposed above the base and surrounded at least partially by a side wall extending distally from the base, where the side wall is configured not to contact a proximal surface of the sensor; a plurality of support ledges projecting radially inward from the side wall and configured to contact the proximal surface of the sensor; and at least one retention feature extending distally from at least one support ledge of the plurality of support ledges and configured to contact a side surface of the sensor.

Implementations may include one or more of the following features. In some embodiments, the spacer is configured to center the sensor within the sensor housing relative to a longitudinal axis of the sensor housing. In some embodiments, the spacer is configured to control a depth of the sensor within the sensor housing along a longitudinal axis of the sensor housing. In some embodiments, the spacer is configured to control an alignment of the sensor relative to a longitudinal axis of the sensor housing. In some embodiments, the spacer includes a first polymer material. In some embodiments, the method further including adhering the sensor to the spacer by a second polymer material positioned at least partially within the recess. In some embodiments, the method further including adhering the spacer to the sensor housing by a third polymer material in contact with a proximal surface of the spacer. In some embodiments, the at least two electrical wires are fixedly attached to and electrically coupled with the sensor. In some embodiments, the at least one retention feature includes a plurality of tabs, where each tab of the plurality of tabs extends distally from a support ledge of the plurality of support ledges. In some embodiments, the retention feature includes the side wall or a counterbore thereof. Implementations of the described techniques may include hardware, a method or process, or computer software on a computer-accessible medium. Other embodiments of this aspect include corresponding computer systems, apparatus, and computer programs recorded on one or more computer storage devices, each configured to perform the actions of the methods.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the flow measurement system, as defined in the claims, is provided in the following written description of various embodiments of the disclosure and illustrated in the accompanying drawings.

### BRIEF DESCRIPTION OF THE DRAWINGS

Illustrative embodiments of the present disclosure will be described with reference to the accompanying drawings, of which:
**Figure 1** is a diagrammatic side view of an intravascular sensing system that includes an intravascular device comprising an a multi-filar electrical conductor bundle, according to aspects of the present disclosure.
**Figure 2** is a perspective view of an example sensor of an intravascular device, in accordance with aspects of the present disclosure.
**Figure 3** is a perspective view of an example sensor assembly 300, in accordance with at least one embodiment of the present disclosure.
**Figure 4** is a perspective view of at least some components of an example sensor assembly, in accordance with at least one embodiment of the present disclosure.
**Figure 5** is a perspective, cross-sectional view of at least a portion of a sensor assembly 300, in accordance with at least one embodiment of the present disclosure.
**Figure 6** is a side cross-sectional view of at least a portion of an example sensor assembly 300, in accordance with at least one embodiment of the present disclosure.
**Figure 7** is a perspective view of an example sensor fitted within an example sensor spacer, in accordance with at least one embodiment of the present disclosure.
**Figure 8** is a perspective view of an example sensor spacer, in accordance with at least one embodiment of the present disclosure.
**Figure 9** is a top view of an example sensor spacer, in accordance with at least one embodiment of the present disclosure.
**Figure 10** is a side cross-sectional view of an example sensor spacer, in accordance with at least one embodiment of the present disclosure.
**Figure 11** is a perspective view of an alternative embodiment of the sensor spacer, in accordance with at least one embodiment of the present disclosure.
**Figure 12** is a perspective, cross-sectional view of the second polymer material of Figure 6, in accordance with at least one embodiment of the present disclosure.
**Figure 13** is a schematic diagram of a processor circuit, in accordance with at least one embodiment of the present disclosure.
**Figure 14** is a schematic view, in flow diagram form, of an example method 1400 for assembling an intraluminal sensing device, in accordance with at least one embodiment of the present disclosure.

### DETAILED DESCRIPTION

Coronary artery disease (CAD) is among the world's leading causes of death. To address this problem, Image Guided Therapy (IGT) imaging systems (for e.g. coronary angiography) and-body diagnostic devices (e.g. pressure-sensing guidewires or intravascular ultrasound catheters) may be used. One such diagnostic device is the blood flow velocity sensing guidewire, which can be used for example to assess Non-Obstructive Coronary Artery Disease (NOCAD) and MicroVascular Disease (MVD). These guidewires are equipped with a single-element ultrasound transducer that is located at its tip. The transducer can emit ultrasound waves in a forward-looking direction and receive the corresponding pulse-echo signals. By pulsed-wave (PW) Doppler analysis, the blood velocity distribution in a specific sampling volume can be deduced.

Existing sensor assemblies may include multiple manual assembly steps that may require high dexterity and skill to perform, even under high magnification. At present, the position of the transducer location within the housing is dependent on the volume and shape of the adhesive backing layers on the proximal face of the transducer. These adhesive volumes may be too small to control consistently, and thus the depth and angle of the transducer relative to the housing may vary. This results in the transducer requiring plasma cleaning and parylene coating to prevent electrical shorts, and difficulties in achieving the specified matching layer thickness, which can require rework of the grinding process. Inconsistency of the matching layer can ultimately result in more variation in sensitivity and electrical performance of the sensor.

The disclosed sensor spacer includes a base to limit the depth of the transducer in the housing, with a through-hole for conductive leads to pass through; a recess to allow a known thickness of backing adhesive to be applied to the transducer; a ledge to seat the transducer, while limiting the contact area between the transducer and spacer to reduce the likelihood of negatively impacting acoustic performance; and, a centering feature to control concentricity between the transducer and housing.

The sensor spacer of the present disclosure may improve the consistency of the transducer location within the housing during assembly. The 3D printing method called 2-Photon Polymerization is known from academic research, and the ability of this method to create parts with sub-micron accuracy lends itself well to an application with tight tolerances.

The present disclosure provides a spacer that fits between a cavity within the sensor housing and the distal surface of the sensor. The sensor spacer (1) limits the depth that the sensor can go within the sensor housing; (2) helps align the sensor such that the distal surface of the sensor is orthogonal to the longitudinal axis of the sensor housing; (3) centers the sensor within the sensor housing; and (4) prevents the proximal end of the sensor from electrically shorting against the conductive material of the sensor housing. This improved sensor assembly design may be thus be significantly more robust than existing systems, with components being harder to damage during assembly, handling, or use. The sensor spacer may for example be producible through single-component or multi-component additive manufacturing (e.g., 3D printing), and may include retention features, to receive a sensing element (e.g., an ultrasound transducer), while minimizing the contact area between the sensor and the sensor spacer (e.g., limited to 5-10% of the surface area of the sensor).

The present disclosure aids substantially in the fabrication and assembly of intraluminal sensing systems. Implemented on an ultrasound guidewire in communication with a processor, the sensor housing or sensor assembly disclosed herein provides a spacer between portions of the sensor and portions of the sensor housing. This improved design transforms a tedious, skill-intensive guidewire assembly process into a process that can be performed in less time, with less knowledge, less training, less manual dexterity, and fewer errors, without the normally routine need to inspect each finished assembly for proper positioning and alignment of the sensor within the sensor housing. This unconventional approach improves the functioning of the flow-sensing guidewire, by reducing the chance of manufacturing errors.

These descriptions are provided for exemplary purposes only, and should not be considered to limit the scope of the sensor spacer.

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. Additionally, while the description below may refer to blood vessels, it will be understood that the present disclosure is not limited to such applications. For example, the devices, systems, and methods described herein may be used in any body chamber or body lumen, including an esophagus, veins, arteries, intestines, ventricles, atria, or any other body lumen and/or chamber. For the sake of brevity, however, the numerous iterations of these combinations will not be described separately.

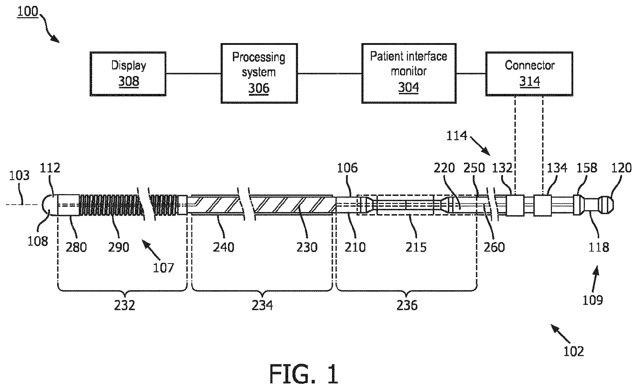

**Figure 1** is a diagrammatic side view of an intravascular sensing system 100 that includes an intravascular device 102 comprising a multi-filar electrical conductor bundle 230, according to aspects of the present disclosure. The intravascular device 102 can be an intravascular guidewire sized and shaped for positioning within a vessel of a patient. The intravascular device 102 can include a distal tip 108 and a sensing component or sensor 112. The sensor 112 can be an electronic sensor, electromechanical sensor, mechanical sensor, optical sensor, and/or other suitable type of sensor. For example, the sensor 112 can be a flow sensor configured to measure the velocity of blood flow within a blood vessel of a patient, a pressure sensor configured to measure a pressure of blood flowing within the vessel, or another type of sensor including but not limited to a temperature or imaging sensor. For example, flow data obtained by a flow sensor can be used to calculate physiological variables such as coronary flow reserve (CFR). Pressure data obtained by a pressure sensor may for example be used to calculate a physiological pressure ratio (e.g., FFR, iFR, Pd/Pa, or any other suitable pressure ratio). An imaging sensor may include an intravascular ultrasound (IVUS), intracardiac echocardiography (ICE), optical coherence tomography (OCT), or intravascular photoacoustic (IVPA) imaging sensor. For example, the imaging sensor can include one or more ultrasound transducer elements, including an array of ultrasound transducer elements.

The intravascular device 102 includes a flexible elongate member 106. The sensor 112 is disposed at the distal portion 107 of the flexible elongate member 106. The sensor 112 can be mounted at the distal portion 107 within a housing 280 in some embodiments. A flexible tip coil 290 extends distally from the housing 280 at the distal portion 107 of the flexible elongate member 106. A connection portion 114 located at a proximal end of the flexible elongate member 106 includes conductive portions 132, 134. In some embodiments, the conductive portions 132, 134 can be conductive ink that is printed and/or deposited around the connection portion 114 of the flexible elongate member 106. In some embodiments, the conductive portions 132, 134 are conductive, metallic rings that are positioned around the flexible elongate member. A locking section is formed by collar 118 and knob 120 are disposed at the proximal portion 109 of the flexible elongate member 106.

The intravascular device 102 in Figure 1 includes a distal core wire 210 and a proximal core wire 220. The distal core 210 and the proximal core 220 are metallic components forming part of the body of the intravascular device 102. For example, the distal core 210 and the proximal core 220 are flexible metallic rods that provide structure for the flexible elongate member 106. The diameter of the distal core 210 and the proximal core 220 can vary along its length. A joint between the distal core 210 and proximal core 220 is surrounded and contained by a hypotube 215.

In some embodiments, the intravascular device 102 comprises a distal assembly and a proximal assembly that are electrically and mechanically joined together, which provides for electrical communication between the sensor 112 and the conductive portions 132, 134. For example, flow data obtained by the sensor 112 (in this example, sensor 112 is a flow sensor) can be transmitted to the conductive portions 132, 134. Control signals (e.g., operating voltage, start/stop commands, etc.) from a processor system 306 in communication with the intravascular device 102 can be transmitted to the sensor 112 via a connector 314 that is attached to the conductive portions 132, 134. The distal subassembly can include the distal core 210. The distal subassembly can also include the sensor 112, the multi-filar conductor bundle 230, and/or one or more layers of insulative polymer/plastic 240 surrounding the conductive members 230 and the core 210. For example, the polymer/plastic layer(s) can insulate and protect the conductive members of the multi-filar cable or conductor bundle 230. The proximal subassembly can include the proximal core 220. The proximal subassembly can also include one or more layers of polymer layer(s) 250 (hereinafter polymer layer 250) surrounding the proximal core 220 and/or conductive ribbons 260 embedded within the one or more insulative and/or protective polymer layer(s) 250. In some embodiments, the proximal subassembly and the distal subassembly can be separately manufactured. During the assembly process for the intravascular device 102, the proximal subassembly and the distal subassembly can be electrically and mechanically joined together. As used herein, flexible elongate member can refer to one or more components along the entire length of the intravascular device 102, one or more components of the proximal subassembly (e.g., including the proximal core 220, etc.), and/or one or more components the distal subassembly (e.g., including the distal core 210, etc.). The joint between the proximal core 220 and distal core 210 is surrounded by the hypotube 215.

In various embodiments, the intravascular device 102 can include one, two, three, or more core wires extending along its length. For example, in one embodiment, a single core wire extends substantially along the entire length of the flexible elongate member 106. In such embodiments, a locking section 118 and a section 120 can be integrally formed at the proximal portion of the single core wire. The sensor 112 can be secured at the distal portion of the single core wire. In other embodiments, such as the embodiment illustrated in Figure 1, the locking section 118 and the section 120 can be integrally formed at the proximal portion of the proximal core 220. The sensor 112 can be secured at the distal portion of the distal core 210. The intravascular device 102 includes one or more conductive members in a multi-filar conductor bundle 230 in communication with the sensor 112. For example, the conductor bundle 230 can include one or more electrical wires that are directly in communication with the sensor 112. In some instances, the conductive members 230 are electrically and mechanically coupled to the sensor 112 by, e.g., soldering. In some instances, the conductor bundle 230 comprises two or three electrical wires (e.g., a bifilar cable or a trifilar cable). An individual electrical wire can include a bare metallic conductor, or a metallic conductor surrounded by one or more insulating layers. The multi-filar conductor bundle 230 can extend along a length of the distal core 210. For example, at least a portion of the conductive members 230 can be helically, or spirally, wrapped around an entire length of the distal core 210, or a portion of the length of the distal core 210.

The intravascular device 102 includes one or more conductive ribbons 260 at the proximal portion of the flexible elongate member 106. The conductive ribbons 260 are embedded within polymer layer(s) 250. The conductive ribbons 260 are directly in communication with the conductive portions 132 and/or 134. In some instances, the multi-filar conductor bundle 230 is electrically and mechanically coupled to the sensor 112 by, e.g., soldering. In some instances, the conductive portions 132 and/or 134 comprise conductive ink (e.g., metallic nano-ink, such as silver or gold nano-ink) that is deposited or printed directed over the conductive ribbons 260.

As described herein, electrical communication between the conductive members 230 and the conductive ribbons 260 can be established at the connection portion 114 of the flexible elongate member 106. By establishing electrical communication between the conductor bundle 230 and the conductive ribbons 260, the conductive portions 132, 134 can be in electrically communication with the sensor 112.

In some embodiments represented by Figure 1, intravascular device 102 includes a locking section 118 and a section 120. To form locking section 118, a machining process is necessary to remove polymer layer 250 and conductive ribbons 260 in locking section 118 and to shape proximal core 220 in locking section 118 to the desired shape. As shown in Figure 1, locking section 118 includes a reduced diameter while section 120 has a diameter substantially similar to that of proximal core 220 in the connection portion 114. In some instances, because the machining process removes conductive ribbons in locking section 118, proximal ends of the conductive ribbons 260 would be exposed to moisture and/or liquids, such as blood, saline solutions, disinfectants, and/or enzyme cleaner solutions, an insulation layer 158 is formed over the proximal end portion of the connection portion 114 to insulate the exposed conductive ribbons.

In some embodiments, a connector 314 provides electrical connectivity between the conductive portions 132, 134 and a patient interface module or patient interface monitor 304. The patient interface monitor (PIM) 304 may in some cases connect to a console or processing system 306, which includes or is in communication with a display 308. In some embodiments, the patient interface monitor 304 includes signal processing circuitry, such as an analog-to-digital converter (ADC), analog and/or digital filters, signal conditioning circuitry, and any other suitable signal processing circuitry for processing the signals provided by the sensor 112 for use by the processing system 306.

The system 100 may be deployed in a catheterization laboratory having a control room. The processing system 306 may be located in the control room. Optionally, the processing system 306 may be located elsewhere, such as in the catheterization laboratory itself. The catheterization laboratory may include a sterile field while its associated control room may or may not be sterile depending on the procedure to be performed and/or on the health care facility. In some embodiments, device 102 may be controlled from a remote location such as the control room, such than an operator is not required to be in close proximity to the patient.

The intraluminal device 102, PIM 304, and display 308 may be communicatively coupled directly or indirectly to the processing system 306. These elements may be communicatively coupled to the medical processing system 306 via a wired connection such as a standard copper multi-filar conductor bundle 230. The processing system 306 may be communicatively coupled to one or more data networks, e.g., a TCP/IP-based local area network (LAN). In other embodiments, different protocols may be utilized such as Synchronous Optical Networking (SONET). In some cases, the processing system 306 may be communicatively coupled to a wide area network (WAN).

The PIM 304 transfers the received signals to the processing system 306 where the information is processed and displayed on the display 308. The console or processing system 306 can include a processor and a memory. The processing system 306 may be operable to facilitate the features of the intravascular sensing system 100 described herein. For example, the processor can execute computer readable instructions stored on the non-transitory tangible computer readable medium.

The PIM 304 facilitates communication of signals between the processing system 306 and the intraluminal device 102. In some embodiments, the PIM 304 performs preliminary processing of data prior to relaying the data to the processing system 306. In examples of such embodiments, the PIM 304 performs amplification, filtering, and/or aggregating of the data. In an embodiment, the PIM 304 also supplies high- and low-voltage DC power to support operation of the intraluminal device 102 via the multi-filar conductor bundle 230.

The multi-filar cable or transmission line bundle 230 can include a plurality of conductors, including one, two, three, four, five, six, seven, or more conductors. The multi-filar conductor bundle 230 can be positioned along the exterior of the distal core 210. The multi-filar conductor bundle 230 and the distal core 210 can be overcoated with an insulative and/or protective polymer 240. In the example shown in Figure 1, the multi-filar conductor bundle 230 includes two straight portions 232 and 236, where the multi-filar conductor bundle 230 extends linearly and parallel to a longitudinal axis 103 of the flexible elongate member 106 on the exterior of the distal core 210, and a helical or spiral portion 234, where the multi-filar conductor bundle 230 is wrapped around the exterior of the distal core 210. In some embodiments, the multi-filar conductor bundle 230 only includes a straight portion or only includes a helical or spiral portion. In general, the multi-filar conductor bundle 230 can extend in a linear, wrapped, non-linear, or non-wrapped manner, or any combination thererof. Communication, if any, along the multi-filar conductor bundle 230 may be through numerous methods or protocols, including serial, parallel, and otherwise, wherein one or more filars of the bundle 230 carry signals. One or more filars of the multi-filar conductor bundle 230 may also carry direct current (DC) power, alternating current (AC) power, or serve as an electrical ground connection.

The display or monitor 308 may be a display device such as a computer monitor, a touch-screen display, a television screen, or any other suitable type of display. The monitor 308 may be used to display selectable prompts, instructions, and visualizations of imaging data to a user. In some embodiments, the monitor 308 may be used to provide a procedurespecific workflow to a user to complete an intraluminal imaging procedure.

Before continuing, it should be noted that the examples described above are provided for purposes of illustration, and are not intended to be limiting. Other devices and/or device configurations may be utilized to carry out the operations described herein.

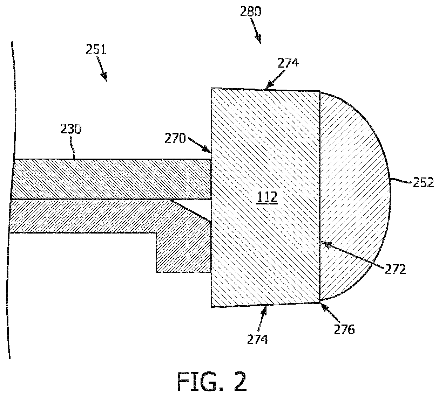

**Figure 2** is a diagrammatic cross-sectional view of an example sensor assembly 251, which may for example be included in the intravascular device 102 of Figure 1. More specifically, Figure 2 illustrates a sensor assembly 251 that includes a sensing component or sensor 112, a housing 280, and an acoustic matching layer 252. As indicated by the positions of the sensing component 112 and the housing 280 illustrated in Figure 1, the sensor assembly 251 may be included in a distal portion of the intravascular device 102 such that the surface 272 of the sensing component 112 faces distally.

As illustrated in Figure 2, the sensing component 112 is positioned within the housing 280 and includes a proximal surface 270, an opposite, distal surface 272, and a side surface 274. In some embodiments, one or more of the proximal surface 270, the distal surface 272, or the side surface 274 may be coated in an insulating layer 276. The insulating layer 276 may be formed from parylene, which may be deposited on the one or more surfaces, for example. The insulating layer 276 may additionally or alternatively be formed from any other suitable insulating material. In some embodiments, the insulating layer 276 may prevent a short (e.g., an electrical failure), which may otherwise be caused by contact between a conductive portion of the sensing component 112 and the housing 280, which may be formed with a metal. As used herein, references to the distal surface 272 encompass the insulating layer 276 in embodiments where a distal end of the sensing component 112 is covered by the insulating layer 276, references to the proximal surface 270 encompass the insulating layer in embodiments where a proximal end of the sensing component 112 is covered by the insulating layer 276, and references to the side surface 274 encompass the insulating layer in embodiments where the side of the sensing component 112 is covered by the insulating layer 276 unless indicated otherwise. As described herein, aspects of the sensor spacer advantageously position the sensor 112 relative to the house 280 so that the chances of shorting are minimized or eliminated. In some aspects, the insulating layer 276 can be omitted as a result of using the sensor spacer described herein.

In some embodiments, the sensing component or sensor 112 may include a transducer element, such as an ultrasound transducer element on the distal surface 272 such that the transducer element faces distally and may be used by the sensing component 112 to obtain sensor data corresponding to a structure distal of the sensing component 112. The sensing component 112 may additionally or alternatively include a transducer element on the proximal surface 270 such that the transducer faces proximally and may be used to obtain sensor data corresponding to a structure proximal of the sensing component. A transducer element may additionally or alternatively be positioned on a side surface 274 (e.g., on a perimeter or circumference) of the sensing component 112 in some embodiments. In some embodiments, a transducer and its associated electrodes and electrical connection points may form the entire sensing component 112, such that all surfaces of the sensing component 112 comprise the transducer.

As further illustrated, the sensing component 112 is coupled to the multi-filar conductor bundle 230, and at least a portion (e.g., a distal portion) of the multi-filar conductor bundle 230 are extends through the housing 280. In some embodiments, the multi-filar conductor bundle 230 and the sensing component 112 may be physically (e.g., mechanically) coupled. Further, one or more filars (e.g., conductive members) of the multi-filar conductor bundle 230 may electrically couple to (e.g., be in electrical communication) with the sensing component 112. In particular, one or more filars of the multi-filar conductor bundle 230 may couple to an element, such as a transducer (e.g., an ultrasound transducer), of the sensing component 112 and may provide power, control signals, an electrical ground or signal return, and/or the like to the element. As described above, such an element may be positioned on the distal surface 272 of the sensor. In that regard, in some embodiments, one or more filars of the multi-filar conductor bundle 230 may extend through a cutout or hole in the sensing component 112 (e.g., in at least the proximal surface 270) to establish electrical communication with an element on the distal surface 272 of the sensor. Filars may additionally or alternatively wrap around the side surface 274 to establish electrical communication with the element on the distal surface 272. Moreover, in some embodiments, filars of the multi-filar conductor bundle 230 may terminate at and/or electrically couple to the proximal surface 270 (e.g., to an element on the proximal surface 270) of the sensing component 112. Further, in some embodiments, a subset of the filars of the multi-filar conductor bundle 230 may extend to the distal surface 272 and/or electrically couple to an element at the distal surface 272, while a different subset of the filars may electrically couple to an element at the proximal surface 270, for example.

In some embodiments, the multi-filar conductor bundle 230 may be coated in the insulating layer 276. In some embodiments, for example, the multi-filar conductor bundle 230 and the sensing component 112 may be coupled together in a sub-assembly before being positioned in the housing 280. In such embodiments, the insulating layer 276 may be applied (e.g., coated and/or deposited) onto the entire sub-assembly, resulting in an insulating layer 276 on both the sensing component 112 and the multi-filar conductor bundle 230.

In some embodiments, the acoustic matching layer 252 may be positioned on (e.g., over) the distal surface 272 of the sensing component 112. In particular, the acoustic matching layer 252 may be disposed directly on the sensing component 112, or the acoustic matching layer 252 may be disposed on the insulating layer 276 coating the sensing component 112. Further, the acoustic matching layer 252 may be disposed on a transducer element (e.g., an ultrasound transducer element) positioned on the sensing component (e.g., the distal surface 272) and/or at least a portion of a conductive filar of the multi-filar conductor bundle 230 that is in communication with the transducer element, such as a filar extending through a hole or along a side of the sensing component 112. To that end, the acoustic matching layer 252 may contact and/or at least partially surround the portion of the conductive filar and/or the transducer element. Moreover, the acoustic matching layer 252 may provide acoustic matching to the sensing component 112 (e.g., to an ultrasound transducer of the sensing component 112). For instance, the acoustic matching layer 252 may minimize acoustic impedance mismatch between the ultrasound transducer and a sensed medium, such as a fluid and/or a lumen that the intravascular device 102 is positioned within. In that regard, the acoustic matching layer 252 may be formed from any suitable material, such as a polymer or an adhesive, to provide acoustic matching with the sensing component 112. The portion of the acoustic matching layer 252 positioned on the distal surface 272 may include and/or be formed from the same material as a portion of the acoustic matching layer positioned on the side surface 274 and/or the proximal surface 270. Further, the acoustic matching layer 252 may be applied to the sensing component 112 before or after the sensing component 112 is positioned within the housing 280 during assembly of the sensor assembly 251. In this regard, the portion of the acoustic matching layer 252 positioned on the distal surface 272 and the portion of the acoustic matching layer positioned on the side surface 274 and/or the proximal surface 270 may be included in the sensor assembly 251 in the same or different steps. Further, in addition to the one or more materials the acoustic matching layer 252 is formed from, the acoustic matching layer 252 may provide acoustic matching with the sensing component 112 via one or more dimensions of the acoustic matching layer 252.

In some embodiments, the sensor assembly 251 may include an atraumatic tip, such as the distal tip 108 illustrated in Figure 1. In some embodiments, the distal tip 108 may include the same material as the acoustic matching layer 252. In some embodiments, the distal tip may include a different material than the acoustic matching layer 252. Additionally or alternatively the distal tip 108 may be formed from one or more layers of materials. The layers may include different materials and/or different configurations (e.g., shape and/or profile, thickness, and/or the like). Further, the distal tip 108 may be arranged to cover the distal surface 272 of the sensing component 112. In some embodiments, the distal tip 108 may also cover a distal end 272 of the housing 280. Moreover, while the distal tip 108 is illustrated as having a domed shape, embodiments are not limited thereto. In this regard, the distal tip 108 may include a flattened profile or any suitable shape. In some embodiments, the entire sensing component 112 may be positioned within (e.g., surrounded by the continuous surface of) the housing 280.

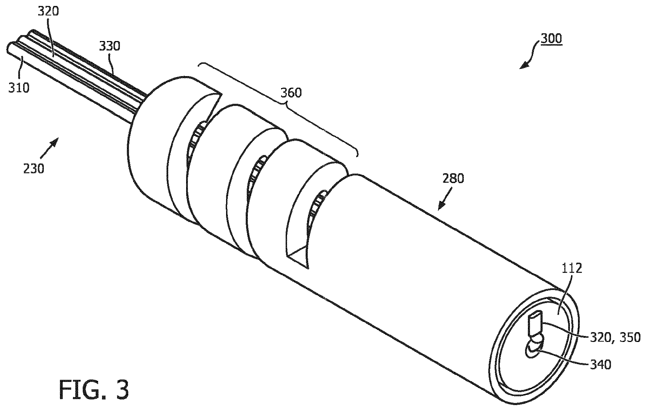

**Figure 3** is a perspective view of an example sensor assembly 300, in accordance with at least one embodiment of the present disclosure. The sensor assembly includes a sensor housing 280, a cable or conductor bundle 230, and a sensor, sensing component, or transducer 112. In the example shown in Figure 3, the sensor housing 280 comprises a spiralcut region 360 that facilitates threading of the coil 290 onto the proximal end of the sensor housing 280 (see Figure 1). The cable or conductor bundle 230 comprises insulated filars, conductors, or wires 310, 320, and 330. Wire 320 extends through a central lumen 340 in the sensor 112 to form a distal connection 350 with the distal face of the sensor 112.

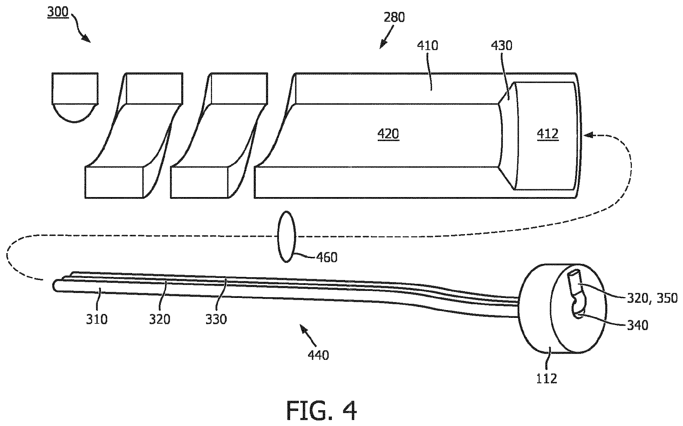

**Figure 4** is a perspective view of at least some components of an example sensor assembly, in accordance with at least one embodiment of the present disclosure. Visible are the sensor housing 280 and a sensor electrical subassembly 440. The sensor housing 280 is formed from a hypotube 410 that includes a central lumen 420. The sensor housing 280 also includes a sensor cavity 412 that connects to the central lumen 420 by a tapered region 430. The sensor electrical subassembly (which may in some cases be referred to colloquially as a "tadpole") includes the electrical conductors 310, 320, and 330, along with the sensor or transducer 112. As seen previously in Figure 3, wire 320 extends through the sensor central lumen 340 to form a distal connection 350 with the distal face of the sensor or transducer 112.

To form the sensor assembly 300 of Figure 3, an assembly technician may for example place a polymer material 460 (e.g., an adhesive or potting material) on the proximal face of the sensor 112 and/or in the sensor cavity 412 of the sensor housing 280. The assembly technician may then pull the wires 310, 320, and 330 through the sensor cavity 412, tapered region 430, and the central lumen 420 of the housing 280 until the sensor 112 is seated within the sensor cavity 412.

During this process, it can be difficult to control the quantity of polymer material 460, the depth of the sensor 112 within the sensor cavity 412, the centering of the sensor 112 within the sensor cavity 412, and the alignment of the sensor 112. Variations in these parameters can lead to corresponding variations in the performance of finished devices. For example, if the sensor 112 is seated too deeply within the sensor cavity 412, then the acoustic matching layer 252 (see Figure 2) may be thicker, resulting in weaker output from the sensor and weaker return echoes into the sensor. Similarly, if the sensor 112 is misaligned within the sensor cavity 412, then the acoustic pulses emitted by the sensor 112 may be equally misaligned, resulting in reduced accuracy in flow measurements. In extreme cases, if the proximal face of the sensor 112 makes contact with the hypotube 410 within the tapered region 430, or if a side face of the sensor 112 makes contact with the hypotube 410 within the sensor cavity 412, the sensor 112 may form an electrical short with the conductive material of the hypotube 410, which may degrade performance of the sensor 112, or prevent the sensor 112 from functioning at all.

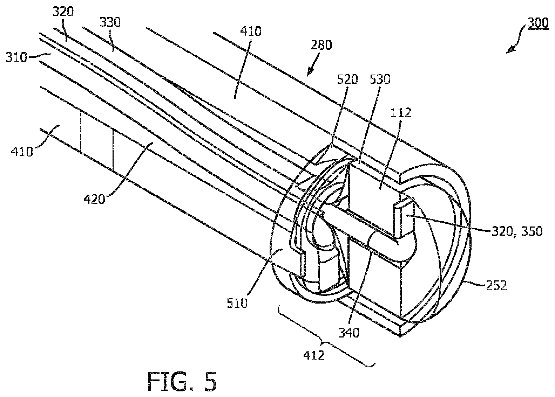

As shown below in Figures 5-12, introduction of a spacer between the sensor 112 and housing 280 can dramatically reduce the risk and/or incidence of these issues.

**Figure 5** is a perspective, cross-sectional view of at least a portion of a sensor assembly 300, in accordance with at least one embodiment of the present disclosure. Visible are the hypotube 410, hypotube central lumen 420, wires 310, 320, and 330, sensor 112, sensor central lumen 340, acoustic matching layer 252, and distal sensor connection 350. However, in the example shown in Figure 5, a sensor spacer 510 is positioned within the sensor cavity 412, between the proximal face of the sensor 112 and the proximal end 520 of the sensor cavity 412. The sensor spacer 510 limits the depth to which the sensor 112 can be pulled into the sensor cavity 412 during the assembly process, and thus prevents the proximal face of the sensor 112 from shorting against the proximal end of the sensor cavity 412. In addition, as shown below, features of the sensor spacer 510 help to center the sensor 112 within the sensor cavity 412, thus preventing the sides of the sensor 112 from shorting against the inner wall 530 of the sensor cavity 412. Since the spacer centers the sensor and prevents physical contact between the sensor and housing, it may then also be possible to eliminate a process in which the sensor/wire assembly is coated with parylene, which is in part for electrical insulation, so similar electrically insulating coatings may be eliminated as well. Furthermore, controlling the depth of the sensor in the housing may result in an improved ability to achieve consistent thickness of the acoustic matching layer on the distal side of the sensor in later manufacturing processes, which could improve or make more consistent the electrical and acoustic performance of the finished product. This improved consistency can reduce or eliminate the need to calibrate individual guidewires to individual connectors, which can advantageously de-risk medical procedures where the surgeon may replace the guidewire without replacing the connector (and thus invalidate or negate the calibration between the guidewire and connector and possibly result in unacceptably high power output to/from the sensor in the body). The sensor spacer 510 may for example be made of a polymer, e.g., a photopolymer material such as Nanoscribe IP-S, or another electrically insulating material, and may be selected to have an acoustic impedance in a range that will reflect or absorb ultrasound energy emitted by the sensor 112, so as not to interfere with the normal acoustic performance of the sensor 112.

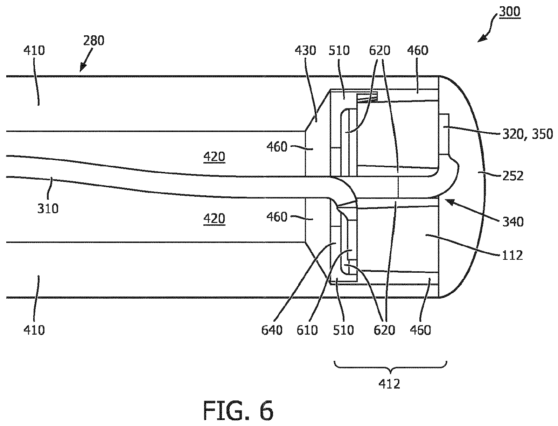

**Figure 6** is a side cross-sectional view of at least a portion of an example sensor assembly 300, in accordance with at least one embodiment of the present disclosure. Visible are the hypotube 410, hypotube central lumen 420, wires 310, 320, and 330, sensor 112, sensor central lumen 340, acoustic matching layer 252, distal sensor connection 350, and sensor spacer 510. Also visible is a proximal sensor connection 610 formed between wire 310 and the proximal face of the sensor 112. The sensor 112 can be a transducer, such as a single transducer element. The sensor 112 can obtain ultrasound data representative of blood flow velocity. In some aspects, the ultrasound data obtained by the sensor 112 does not provide an image of a blood vessel. In an example, the sensor 112 is made of a piezoelectric material such as lead zirconate titanate (PZT), such that when a voltage is applied between the distal sensor connection 350 and the proximal sensor connection 610, the piezoelectric material compresses, and when the voltage is released, the piezoelectric material expands. This expansion can generate an acoustic pulse. Similarly, when an acoustic wave impinging on the sensor electronic element 112 briefly compresses the piezoelectric material, a voltage is generated between the distal sensor connection 350 and the proximal sensor connection 610. In this way, the sensor electronic element 112 can send and receive pulses of ultrasound energy. However, it is understood that the sensor electronic element may be of a different sort, including but not limited to a capacitive micromachined ultrasound transducer (CMUT), a piezoelectric micromachined ultrasound transducer (PMUT), an optical transceiver, or otherwise.

The sensor spacer may for example be made of a first polymer material. The sensor 112 is held in place within the sensor spacer 510 by a second polymer material 620 such as an adhesive or potting compound. The second polymer material may be or include an acoustic backing material, e.g., Nuvasil acoustic backing adhesive, although other example materials may include, but are not limited to Dymax 1184, Dymax 9001, or similar photopolymers, parylene, etc, and may for example be selected to have an acoustic impedance within a range that will reflect or absorb ultrasound energy emitted by the sensor electronic element 112, so as not to interfere with the operation of the sensor electronic element 112. In general, an acoustic backing material attenuates ultrasound energy, preventing ultrasound propagation in undesired directions (e.g., proximally from the sensor in this example). In some embodiments, the second polymer material 620 may be chosen for mechanical and adhesive properties, since it may used to strain relieve the wires or conductive leads 310, 320 and fix the transducer assembly (e.g., the sensor electronic element 112 and spacer 510) into the housing 280, and may also be selected to be a suitable acoustic matching layer for the distal side of the transducer or sensor electronic element 112. In the example shown in Figure 6, the second polymer material 620 fills a space between the sensor electronic element 112 and the sensor spacer 510, as well as filling a portion of the sensor central lumen 340 that is not occupied by wire 320.

A third polymer material 460 contacts the proximal face 640 of the sensor spacer 510 and at least partially fills the tapered region 430 of the hypotube 410, in order to adhere the sensor spacer 510 to the sensor housing 280. In the example shown in Figure 6, the third polymer material 460 also at least partially fills the space between the sides of the sensor element 112 and the inner wall of the sensor cavity 412. Example materials may include, but are not limited to Loctite Nuva-Sil silicone potting compound or other potting compounds, and may for example be selected to have an acoustic damping effect for the ultrasound energy emitted by the sensor electronic element 112, so as not to interfere with the operation of the sensor electronic element 112. Depending on the implementation, the third polymer material 460 may be the same or different than the second polymer material 620, and may be the same or different than the material of the acoustic matching layer 252. In general, an acoustic matching layer is used to facilitate ultrasound propagation in the desired direction (distally from sensor in this example).

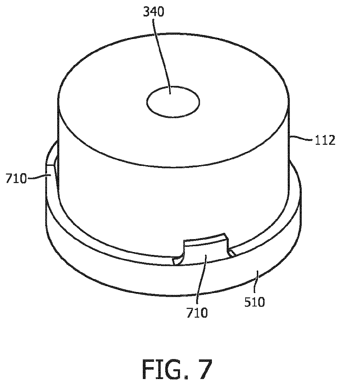

**Figure 7** is a perspective view of an example sensor electronic element 112 fitted within an example sensor spacer 510, in accordance with at least one embodiment of the present disclosure. Visible are the sensor or sensor electronic element 112, the sensor spacer 510, and the sensor central lumen 340. In the example shown in Figure 7, the sensor spacer 510 includes three retention tabs 710 spaced evenly around the circumference of the sensor spacer 510. The retention tabs 710 extend vertically along a portion of the sides of the sensor electronic element 112, in order to center the sensor electronic element 112 within the sensor spacer 510, and thus within the sensor cavity of the sensor housing as well.

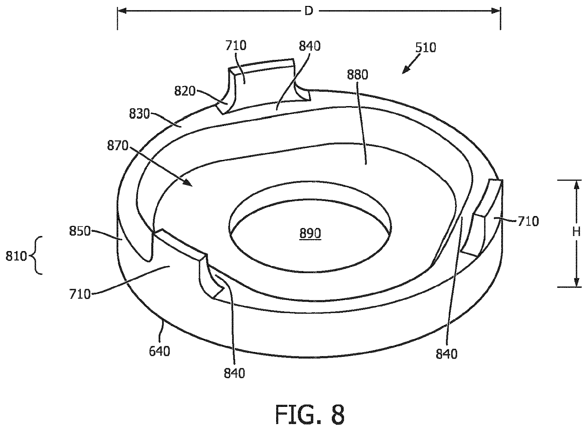

**Figure 8** is a perspective view of an example sensor spacer 510, in accordance with at least one embodiment of the present disclosure. The disclosed sensor spacer 510 includes a base 810 to limit the depth of the transducer in the housing, with a through-hole 890 for wire 320 to pass through (see Figure 6); a recess 870 to allow a known thickness of backing adhesive to be applied to the transducer; a plurality of support ledges 840 to seat the transducer, while limiting the contact area between the transducer and spacer to reduce the likelihood of negatively impacting acoustic performance; and the tabs 710, which serve as a centering feature to control concentricity between the transducer and housing.

A 3D printing method called 2-photon polymerization is known to be capable of creating parts with sub-micron accuracy. Thus, the sensor spacer 510 may be made at least in part from a cured photopolymer formed on a 2-Photon Polymerization 3D printer (e.g. Nanoscribe GT2 or UpNano NanoOne), but could also be produced for example on a high resolution ultraviolet digital light processing (UV DLP) 3D printer (e.g. Boston Micro Fabrication microArch 230), or by micromachining or other semiconductor fabrication techniques, or by other means. For example, the sensor spacer 510 could be made from alternate materials and at higher volumes if made via micro-injection molding. Such a sensor spacer 510 could be implemented in other small ultrasound devices to improve consistency and manufacturability without negatively impacting performance.

Some existing sensor assemblies include a polymer material (e.g., an adhesive) around the sensor 112. Once cured, this polymer material may be solid. However, the precise shape and dimensions of this cured mass of adhesive is difficult to control, thus leading to inconsistency in the depth, centering, and alignment of the sensor 112 within the sensor housing 280. The sensor spacer 510 is different from this technique at least in that it is fabricated as a solid object with sub-micron accuracy.

In the example shown in Figure 8, the tabs 710 include a stress-relieving chamfered region 820 at the lower corners. The tabs 710 project vertically from the rim 830 of a sidewall 850. Widened portions of the rim 830 form the support ledges 840. The sensor spacer 510 is configured such that the sensor 112 (see Figure 7) rests on the support ledges 840 and is centered by the retention tabs 710, without contacting other portions of the rim 830. Thus, contact between the sensor 112 and the sensor spacer 510 may be limited to just 5-10% of the surface area of the sensor. This may limit the effect of the sensor spacer 510 on the acoustic performance of the sensor.

In an example, the floor or distal surface 880 of the recess 870 (which is also the top or distal surface of the base 810) is in contact with the backing adhesive 620 (see Figure 6), which is also in contact with the proximal surface of the sensor 112, to secure the sensor 112 within the sensor spacer 510. Similarly, the bottom surface or proximal surface 640 of the sensor spacer 510 is in contact with adhesive 460 (see Figure 6), to secure the sensor spacer 510 within the sensor housing 280 (see Figure 6).

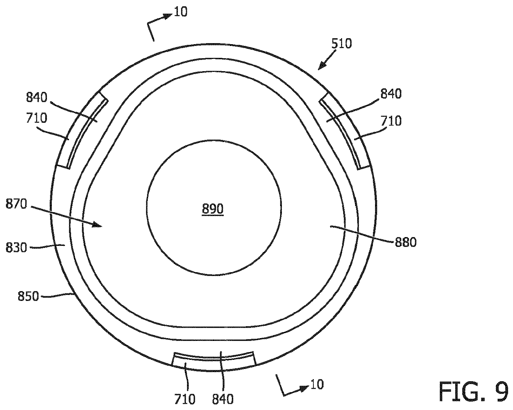

**Figure 9** is a top view of an example sensor spacer 510, in accordance with at least one embodiment of the present disclosure. Visible are the tabs 710, rim 830 of the sidewall 850, support ledges 840, backing adhesive recess 870, recess floor 880, and through-hole 890. In the example shown in Figure 9, the recess 870 has a generally triangular shape, such that the support ledges 840 are radially inward of other portions of the sidewall rim 830. Thus, the diameter D of the sensor spacer 510 (see Figure 8) can be selected such that the sensor electronic element 112( see Figure 7) is supported by the support ledges 840 but is not in contact with other portions of the sidewall rim 830. It is understood that other shapes for the recess 870 and/or other numbers, sizes, or shapes of tabs 710 may be used instead of or in addition to those shown in Figure 9.

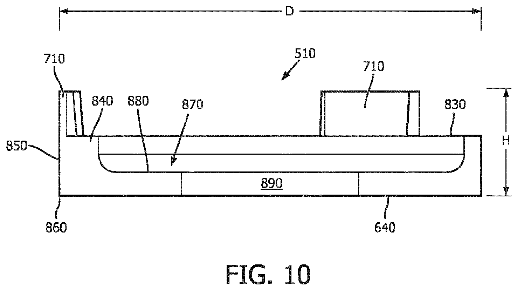

**Figure 10** is a side cross-sectional view of an example sensor spacer 510 along section line 10-10 of Figure 9, in accordance with at least one embodiment of the present disclosure. Visible are the tabs 710, rim 830 of the sidewall 850, support ledges 840, backing adhesive recess 870, recess floor 880, through-hole 890, and bottom surface or proximal surface 640. In an example, a diameter D of the sensor spacer 510 is between 250 and 350 microns ± 1 micron, and a height H of the sensor spacer is between 50 and 100 microns ± 1 micron, although other heights and diameters, both larger and smaller, may be used instead or in addition. In an example, the diameter D may be selected to be approximately 5 microns less than the nominal width of the sensor cavity 412, thus leaving a circumferential gap of approximately 2.5 microns between the sensor spacer 510 and the sensor cavity 412, although other gap sized. Both larger and smaller, may be used instead or in addition. Also important are the longitudinal distance between the proximal face 640 of the sensor spacer 510 and the support ledges 840, as this controls the depth of the transducer or sensor electronic element within the sensor housing.

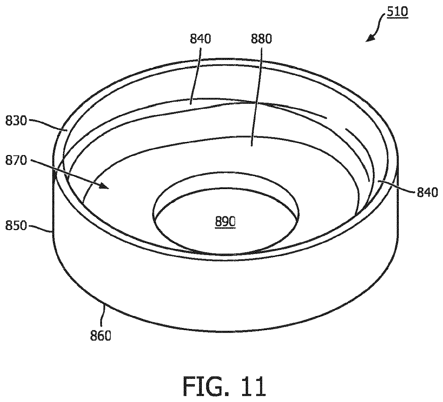

**Figure 11** is a perspective view of an alternative embodiment of the sensor spacer 510, in accordance with at least one embodiment of the present disclosure. In the example shown in Figure 11, the tabs are not present, and the rim 830 of the sidewall 850 has been raised to the height of the tabs, or to a different height sufficient to retain and center the sensor 112 (see Figure 7), while keeping the contact area between the sensor spacer 510 and the sensor 112 below a desired threshold (e.g., below 5% or below 10% of the surface area of the sensor 112). Also visible are the recess 870, recess floor 880, through-hole 890, and bottom surface or proximal surface 860. In some embodiments, the retention feature may be a counterbore of the sidewall, rather than the sidewall itself. For example, a distal portion of the sidewall may be bored out in order to thin the sidewall, and the thinned portion of the sidewall may serve as a retention feature. Other retention features that contact the sides of the sensor 112 may be used instead or in addition.

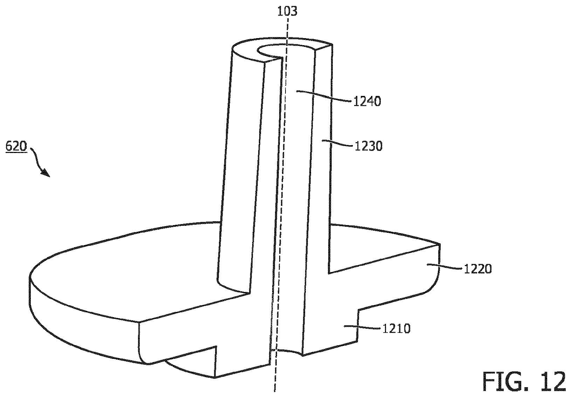

**Figure 12** is a perspective, cross-sectional view of the second polymer material 620 of Figure 6, in accordance with at least one embodiment of the present disclosure. When the second polymer material 620 is introduced between the sensor spacer 510 and the sensor 112 (see Figure 6), the second polymer material 620 may flow between the sensor spacer 510 and the sensor 112 such that a first portion 1210 of the second polymer material 620 fills the through-hole 890 of the sensor spacer 510 (see Figure 8), a second portion 1220 of the second polymer material 620 fills the recess 870 of the sensor spacer 510 (see Figure 8), and a third portion 1230 of the second polymer material 620 fills the central lumen 340 of the sensor 112 (see Figure 7). A through-hole along the longitudinal axis 103 (see Figure 1) of the intraluminal device and of the sensor housing is formed through the first region 1210, second region 1220, and third region 1230 of the second polymer material 620 as the second polymer material flows around wire 320 (see Figure 6).

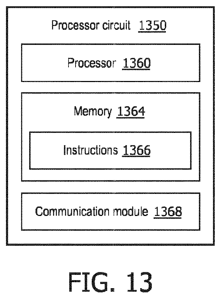

**Figure 13** is a schematic diagram of a processor circuit 1350, in accordance with at least one embodiment of the present disclosure. The processor circuit 1350 may be implemented in the intravascular sensing system 100, processing system 306, or other devices or workstations (e.g., third-party workstations, network routers, etc.), or on a cloud processor or other remote processing unit, as necessary to implement the devices, systems, and methods disclosed herein. As shown, the processor circuit 1350 may include a processor 1360, a memory 1364, and a communication module 1368. These elements may be in direct or indirect communication with each other, for example via one or more buses.

The processor 1360 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1360 may also comprise another hardware device, a firmware device, or any combination thereof configured to perform the operations described herein. The processor 1360 may also be implemented as a combination of computing devices, e.g., a combination of a DSP and a microprocessor, a plurality of microprocessors, one or more microprocessors in conjunction with a DSP core, or any other such configuration.

The memory 1364 may include a cache memory (e.g., a cache memory of the processor 1360), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1364 includes a non-transitory computer-readable medium. The memory 1364 may store instructions 1366. The instructions 1366 may include instructions that, when executed by the processor 1360, cause the processor 1360 to perform the operations described herein. Instructions 1366 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1368 can include any electronic circuitry and/or logic circuitry to facilitate direct or indirect communication of data between the processor circuit 1350, and other processors or devices. In that regard, the communication module 1368 can be an input/output (I/O) device. In some instances, the communication module 1368 facilitates direct or indirect communication between various elements of the processor circuit 1350 and/or the intravascular measurement system 100. The communication module 1368 may communicate within the processor circuit 1350 through numerous methods or protocols. Serial communication protocols may include but are not limited to US SPI, I²C, RS-232, RS-485, CAN, Ethernet, ARINC 429, MODBUS, MIL-STD-1553, or any other suitable method or protocol. Parallel protocols include but are not limited to ISA, ATA, SCSI, PCI, IEEE-488, IEEE-1284, and other suitable protocols. Where appropriate, serial and parallel communications may be bridged by a UART, USART, or other appropriate subsystem.

External communication (including but not limited to software updates, firmware updates, preset sharing between the processor and central server, or readings from the ultrasound device) may be accomplished using any suitable wireless or wired communication technology, such as a cable interface such as a USB, micro USB, Lightning, or FireWire interface, Bluetooth, Wi-Fi, ZigBee, Li-Fi, or cellular data connections such as 2G/GSM, 3G/UMTS, 4G/LTE/WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

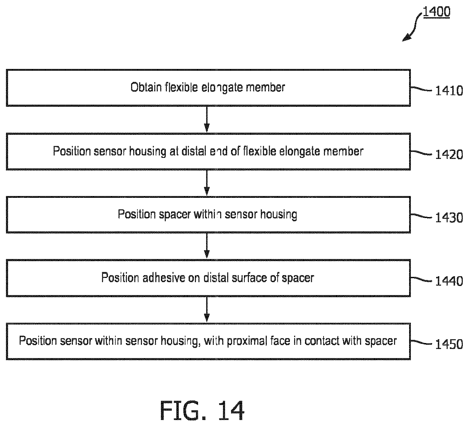

**Figure 14** is a schematic view, in flow diagram form, of an example method 1400 for assembling an intraluminal sensing device, in accordance with at least one embodiment of the present disclosure. It is understood that the steps of method 1400 may be performed in a different order than shown in Figure 14, additional steps can be provided before, during, or after the steps, and/or some of the steps described can be replaced or eliminated in other embodiments. Flow diagrams and block diagrams are provided herein for exemplary purposes; a person of ordinary skill in the art will recognize myriad variations that nonetheless fall within the scope of the present disclosure.

In step 1410, the method includes obtaining a flexible elongate member such as a catheter or guidewire configured for insertion into a body lumen of a patient.

In step 1420, the method includes positioning a sensor housing at or near the distal end of the flexible elongate member.

In step 1430, the method includes positioning a sensor spacer 510 (see Figure 8) within the sensor housing as described above.

In step 1440, the method includes positioning a polymer material such as an adhesive or potting compound on a distal surface of the sensor spacer, as described above.

In step 1450, the method includes positioning a sensor within the sensor housing, such that a proximal surface of the sensor is in contact with the sensor spacer.

Accordingly, it can be seen that the present disclosure improves the operation and assembly process of flow-sensing guidewire devices and systems, by providing a component to center, align, and control the depth of the sensor within the sensor housing, while preventing the sensor from electrically shorting against the conductive material of the sensor housing.

The present disclosure may for example implemented for a flow-sensing guidewire, such as the Philips FloWire (Doppler guide wire) or the Philips ComboWire^{™} that provides simultaneous pressure and flow information. It can also be applied to new flow modalities under development, both for existing devices and for devices hereinafter developed, either with single transducers or multiple transducers, and comprising either a flow-only sensor or a flow sensor combined with a pressure sensor, or with other sensing modalities.

This spacer may be detected non-destructively by use of a high resolution CT scan, or destructively by precision cross-sectioning of the device and viewing the cross section of the sensor area under microscope. Due to the small size and location within the housing, the sensor is unlikely to be detectable by less technical methods. The sensor spacer may be incorporated into devices such as the flow-sensing guidewires, other guide wires (e.g., pressure-sensing guidewires), or other small-scale transducer applications or ultrasound devices.

A number of variations are possible on the examples and embodiments described above. For example, one or more surfaces of the sensor spacer may include coatings such as hydrophobic, hydrophilic, anti-scratch, or other coatings. The surface of the sensor spacer may be plasma treated to increase adhesion. Some components shown as conductive may also function if made from insulators, and vice-versa. The polymer material 620 shown in Figures 6 and 12 may be introduced as a curable liquid or gel, or may be a solid material component fitted into place as part of the assembly process. Adhesives or potting compounds may be selected based on their mechanical, electrical, or acoustic properties. The acoustic matching layer may be replaced with an optically transparent or translucent material, or a material transparent to other types of radiation, the acoustic backing layer may be replaced with an optically reflective or optically absorptive material, or a material reflective or absorptive to other types of radiation, and the sensor may be replaced with an optical emitter, optical sensor, or other type of sensor.

The logical operations making up the embodiments of the technology described herein are referred to variously as operations, steps, objects, elements, components, or modules. It should be understood that these may be arranged or performed in any order, unless explicitly claimed otherwise or a specific order or arrangement is inherently necessitated by the claim language. It should further be understood that the described technology may be employed in single-use and multi-use electrical and electronic devices for medical or nonmedical use.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the sensor spacer. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary embodiments of the sensor spacer as defined in the claims, which define the present invention. Although various embodiments of the claimed subject matter have been described above with a certain degree of particularity, or with reference to one or more individual embodiments, those skilled in the art could make numerous alterations to the disclosed embodiments without departing from the scope of the claimed subject matter

Still other embodiments are contemplated. It is intended that all matter contained in the above description and shown in the accompanying drawings shall be interpreted as illustrative only of particular embodiments and not limiting. Changes in detail or structure may be made without departing from the subject matter as defined in the following claims.

## Claims

1. An intraluminal device (102), comprising:
a flexible elongate member (106) configured to extend in a longitudinal direction within a body lumen of a patient;
a sensor (112) disposed at a distal region of the flexible elongate member, wherein the sensor is configured obtain intraluminal data associated with the body lumen;
a housing (280) at least partially surrounding the sensor; and
a spacer (510) disposed between a portion of the sensor housing and a proximal face of the sensor, wherein the spacer comprises:
a base (810);
a through-hole (890) extending through the base; and
a recess (870) disposed distal of the base and surrounded at least partially by a side wall (850) extending distally from the base, wherein the side wall is configured not to contact a proximal surface of the sensor;
**characterised in that** the spacer further comprises a plurality of support ledges (840) projecting radially inward from the side wall and configured to contact a proximal surface of the sensor.

2. The intraluminal device of claim 1, wherein the spacer comprises at least one retention feature (710) extending distally from at least one support ledge of the plurality of support ledges and configured to contact a side surface of the sensor.

3. The intraluminal device of claim 1 or 2, wherein the spacer is configured to center the sensor within the sensor housing relative to a longitudinal axis of the sensor housing.

4. The intraluminal device of any one of claims 1 to 3, wherein the spacer is configured to control a depth of the sensor within the sensor housing along a longitudinal axis of the sensor housing or an alignment of the sensor relative to a longitudinal axis of the sensor housing.

5. The intraluminal device of any one of claims 1 to 4, wherein the spacer comprises a first polymer material.

6. The intraluminal device of any one of claims 1 to 5, wherein the sensor is adhered to the spacer by a second polymer material positioned at least partially within the recess.

7. The intraluminal device of any one of claims 1 to 6, wherein the spacer is adhered to the sensor housing by a third polymer material in contact with a proximal surface of the spacer.

8. The intraluminal device of claim 2, wherein the at least one retention feature comprises a plurality of tabs, wherein each tab of the plurality of tabs extends distally from a support ledge of the plurality of support ledges.

9. The intraluminal device of claim 2 or 8, wherein the at least one retention feature comprises the side wall or a counterbore thereof.

10. The intraluminal device of any one of the previous claims wherein the intraluminal device is an intravascular device (102) and the sensor comprises a transducer element.

11. The intraluminal device of any one of the previous claims wherein the spacer is producible through single-component or multi-component additive manufacturing.

12. A method for assembling an intraluminal device (102), the method comprising:
obtaining (1410) a flexible elongate member configured to extend in a longitudinal direction within a body lumen of a patient;
positioning (1420) a sensor housing at a distal region of the flexible elongate member;
positioning (1430) a spacer within the sensor housing; and
positioning (1450) a sensor at least partially within the sensor housing distal of the spacer, wherein the sensor is configured obtain intraluminal data associated with the body lumen, wherein the spacer comprises:
a base;
a through-hole extending through the base; and
a recess disposed above the base and surrounded at least partially by a side wall extending distally from the base, wherein the side wall is configured not to contact a proximal surface of the sensor;
**characterised in that** the spacer further comprises a plurality of support ledges projecting radially inward from the side wall and configured to contact the proximal surface of the sensor.

13. The method of claim 12, wherein the spacer further comprises at least one retention feature extending distally from at least one support ledge of the plurality of support ledges and configured to contact a side surface of the sensor, wherein the spacer is configured to center the sensor within the sensor housing relative to a longitudinal axis of the sensor housing, and wherein the spacer is configured to control a depth of the sensor within the sensor housing along a longitudinal axis of the sensor housing or an alignment of the sensor relative to a longitudinal axis of the sensor housing.

14. The method of claim 12 or 13, wherein the spacer comprises a first polymer material and the method further comprising adhering (1440) the sensor to the spacer by a second polymer material positioned at least partially within the recess and adhering the spacer to the sensor housing by a third polymer material in contact with the proximal surface of the spacer.

15. The method of any one of the claims 12 to 14, further wherein the spacer is produced through single-component or multi-component additive manufacturing.

## Patentansprüche

1. Intraluminale Vorrichtung (102), umfassend:
ein flexibles, längliches Element (106), das konfiguriert ist, um sich in Längsrichtung innerhalb eines Körperlumens eines Patienten zu erstrecken;
einen Sensor (112), der an einem distalen Bereich des flexiblen, länglichen Elements angeordnet ist, wobei der Sensor konfiguriert ist, um intraluminale Daten zu erhalten, die mit dem Körperlumen in Zusammenhang stehen;
ein Gehäuse (280), das den Sensor mindestens teilweise umgibt; und
einen Abstandshalter (510), der zwischen einem Abschnitt des Sensorgehäuses und einer proximalen Fläche des Sensors angeordnet ist, wobei der Abstandshalter umfasst:
einen Sockel (810);
ein Durchgangsloch (890), das sich durch den Sockel erstreckt; und
eine Aussparung (870), die distal von dem Sockel angeordnet und mindestens teilweise von einer Seitenwand (850) umgeben ist, die sich distal von dem Sockel aus erstreckt, wobei die Seitenwand so konfiguriert ist, dass sie eine proximale Oberfläche des Sensors nicht berührt;
**dadurch gekennzeichnet, dass** der Abstandshalter ferner eine Vielzahl von Auflagekanten (840) umfasst, die von der Seitenwand radial nach innen ragen und so konfiguriert sind, dass sie eine proximale Oberfläche des Sensors berühren.

2. Intraluminale Vorrichtung nach Anspruch 1, wobei der Abstandshalter mindestens ein Halteelement (710) umfasst, das sich von mindestens einer Auflagekante der Vielzahl von Auflagekanten distal erstreckt und so konfiguriert ist, dass es eine Seitenfläche des Sensors berührt.

3. Intraluminale Vorrichtung nach Anspruch 1 oder 2, wobei der Abstandshalter so konfiguriert ist, dass er den Sensor innerhalb des Sensorgehäuses relativ zu einer Längsachse des Sensorgehäuses zentriert.

4. Intraluminale Vorrichtung nach einem der Ansprüche 1 bis 3, wobei der Abstandshalter so konfiguriert ist, dass er eine Tiefe des Sensors innerhalb des Sensorgehäuses entlang einer Längsachse des Sensorgehäuses oder eine Ausrichtung des Sensors relativ zu einer Längsachse des Sensorgehäuses steuert.

5. Intraluminale Vorrichtung nach einem der Ansprüche 1 bis 4, wobei der Abstandshalter ein erstes Polymermaterial umfasst.

6. Intraluminale Vorrichtung nach einem der Ansprüche 1 bis 5, wobei der Sensor mittels eines zweiten Polymermaterials, das sich mindestens teilweise innerhalb der Aussparung befindet, an dem Abstandshalter angebracht ist.

7. Intraluminale Vorrichtung nach einem der Ansprüche 1 bis 6, wobei der Abstandshalter mittels eines dritten Polymermaterials, das mit einer proximalen Oberfläche des Abstandshalters in Kontakt steht, an dem Sensorgehäuse angebracht ist.

8. Intraluminale Vorrichtung nach Anspruch 2, wobei das mindestens eine Halteelement eine Vielzahl von Laschen umfasst, wobei sich jede Lasche der Vielzahl von Laschen distal von einer Auflagekante der Vielzahl von Auflagekanten erstreckt.

9. Intraluminale Vorrichtung nach Anspruch 2 oder 8, wobei das mindestens eine Halteelement die Seitenwand oder eine Senkung davon umfasst.

10. Intraluminale Vorrichtung nach einem der vorstehenden Ansprüche, wobei es sich bei der intraluminalen Vorrichtung um eine intravaskuläre Vorrichtung (102) handelt und der Sensor ein Wandlerelement umfasst.

11. Intraluminale Vorrichtung nach einem der vorstehenden Ansprüche, wobei der Abstandshalter durch additive Fertigung mit einer oder mehreren Komponenten hergestellt werden kann.

12. Verfahren zur Montage einer intraluminalen Vorrichtung (102), wobei das Verfahren umfasst:
Erhalten (1410) eines flexiblen, länglichen Elements, das konfiguriert ist, um sich in Längsrichtung innerhalb eines Körperlumens eines Patienten zu erstrecken;
Positionieren (1420) eines Sensorgehäuses an einem distalen Bereich des flexiblen, länglichen Elements;
Positionieren (1430) eines Abstandshalters innerhalb des Sensorgehäuses; und
Positionieren (1450) eines Sensors mindestens teilweise innerhalb des Sensorgehäuses distal zum Abstandhalter, wobei der Sensor konfiguriert ist, um intraluminale Daten zu erhalten, die mit dem Körperlumen in Zusammenhang stehen, wobei der Abstandhalter umfasst:
einen Sockel;
ein Durchgangsloch, das sich durch den Sockel erstreckt; und
eine Aussparung, die oberhalb des Sockels angeordnet und mindestens teilweise von einer Seitenwand umgeben ist, die sich distal von dem Sockel aus erstreckt, wobei die Seitenwand so konfiguriert ist, dass sie eine proximale Oberfläche des Sensors nicht berührt;
**dadurch gekennzeichnet, dass** der Abstandshalter ferner eine Vielzahl von Auflagekanten umfasst, die von der Seitenwand radial nach innen ragen und so konfiguriert sind, dass sie die proximale Oberfläche des Sensors berühren.

13. Verfahren nach Anspruch 12, wobei der Abstandshalter ferner mindestens ein Halteelement umfasst, das sich von mindestens einer Auflagekante der Vielzahl von Auflagekanten distal erstreckt und so konfiguriert ist, dass es eine Seitenfläche des Sensors berührt, wobei der Abstandshalter so konfiguriert ist, dass er den Sensor innerhalb des Sensorgehäuses relativ zu einer Längsachse des Sensorgehäuses zentriert, und wobei der Abstandshalter so konfiguriert ist, dass er eine Tiefe des Sensors innerhalb des Sensorgehäuses entlang einer Längsachse des Sensorgehäuses oder eine Ausrichtung des Sensors relativ zu einer Längsachse des Sensorgehäuses steuert.

14. Verfahren nach Anspruch 12 oder 13, wobei der Abstandshalter ein erstes Polymermaterial umfasst und das Verfahren ferner das Anbringen (1440) des Sensors an dem Abstandshalter mittels eines zweiten Polymermaterials, das sich mindestens teilweise innerhalb der Aussparung befindet, und das Anbringen des Abstandshalters an dem Sensorgehäuse mittels eines dritten Polymermaterials, das mit der proximalen Oberfläche des Abstandshalters in Kontakt steht, umfasst.

15. Verfahren nach einem der Ansprüche 12 bis 14, wobei der Abstandshalter durch additive Fertigung mit einer oder mehreren Komponenten hergestellt wird.

## Revendications

1. Dispositif intraluminal (102), comprenant :
un élément allongé flexible (106) configuré pour s'étendre dans une direction longitudinale à l'intérieur d'une lumière corporelle d'un patient ;
un capteur (112) disposé dans une région distale de l'élément allongé flexible, dans lequel le capteur est configuré pour obtenir des données intraluminales associées à la lumière corporelle ;
un boîtier (280) entourant au moins partiellement le capteur ; et
une entretoise (510) disposée entre une partie du boîtier du capteur et une face proximale du capteur, dans lequel l'entretoise comprend :
une base (810) ;
un trou traversant (890) s'étendant à travers la base ; et
un évidement (870) disposé distalement par rapport à la base et entouré au moins partiellement par une paroi latérale (850) s'étendant distalement à partir de la base, dans laquelle la paroi latérale est configurée pour ne pas entrer en contact avec une surface proximale du capteur ;
**caractérisé en ce que** l'entretoise comprend en outre une pluralité de rebords de support (840) faisant saillie radialement vers l'intérieur à partir de la paroi latérale et configurés pour entrer en contact avec une surface proximale du capteur.

2. Dispositif intraluminal selon la revendication 1, dans lequel l'entretoise comprend au moins un élément de rétention (710) s'étendant distalement à partir d'au moins un rebord de support de la pluralité de rebords de support et configuré pour entrer en contact avec une surface latérale du capteur.

3. Dispositif intraluminal selon la revendication 1 ou 2, dans lequel l'entretoise est configurée pour centrer le capteur à l'intérieur du boîtier du capteur par rapport à un axe longitudinal du boîtier du capteur.

4. Dispositif intraluminal selon l'une quelconque des revendications 1 à 3, dans lequel l'entretoise est configurée pour commander une profondeur du capteur à l'intérieur du boîtier du capteur le long d'un axe longitudinal du boîtier du capteur ou un alignement du capteur par rapport à un axe longitudinal du boîtier du capteur.

5. Dispositif intraluminal selon l'une quelconque des revendications 1 à 4, dans lequel l'entretoise comprend un premier matériau polymère.

6. Dispositif intraluminal selon l'une quelconque des revendications 1 à 5, dans lequel le capteur est collé à l'entretoise par un deuxième matériau polymère positionné au moins partiellement à l'intérieur du renfoncement.

7. Dispositif intraluminal selon l'une quelconque des revendications 1 à 6, dans lequel l'entretoise est collée au boîtier du capteur par un troisième matériau polymère en contact avec une surface proximale de l'entretoise.

8. Dispositif intraluminal selon la revendication 2, dans lequel au moins un élément de rétention comprend une pluralité de languettes, dans lequel chaque languette de la pluralité de languettes s'étend distalement à partir d'un rebord de support de la pluralité de rebords de support.

9. Dispositif intraluminal selon la revendication 2 ou 8, dans lequel au moins un élément de rétention comprend la paroi latérale ou un contre-alésage de celle-ci.

10. Dispositif intraluminal selon l'une quelconque des revendications précédentes dans lequel le dispositif intraluminal est un dispositif intravasculaire (102) et le capteur comprend un élément transducteur.

11. Dispositif intraluminal selon l'une quelconque des revendications précédentes, dans lequel l'entretoise est réalisable par fabrication additive monocomposante ou multicomposante.

12. Procédé d'assemblage d'un dispositif intraluminal (102), le procédé comprenant :
l'obtention (1410) d'un élément allongé flexible configuré pour s'étendre dans une direction longitudinale à l'intérieur d'une lumière corporelle d'un patient ;
le positionnement (1420) d'un boîtier de capteur dans une région distale de l'élément allongé flexible ;
le positionnement (1430) d'une entretoise à l'intérieur du boîtier du capteur ; et
le positionnement (1450) d'un capteur au moins partiellement à l'intérieur du boîtier du capteur de manière distale par rapport à l'entretoise, dans lequel le capteur est configuré pour obtenir des données intraluminales associées à la lumière corporelle, dans lequel l'entretoise comprend :
une base ;
un trou traversant s'étendant à travers la base ; et
un évidement disposé au-dessus de la base et entouré au moins partiellement par une paroi latérale s'étendant distalement à partir de la base, dans laquelle la paroi latérale est configurée pour ne pas entrer en contact avec une surface proximale du capteur ;
**caractérisé en ce que** l'entretoise comprend en outre une pluralité de rebords de support faisant saillie radialement vers l'intérieur depuis la paroi latérale et configurés pour entrer en contact avec la surface proximale du capteur.

13. Procédé selon la revendication 12, dans lequel l'entretoise comprend en outre au moins un élément de retenue s'étendant distalement à partir d'au moins un rebord de support parmi la pluralité de rebords de support et configuré pour entrer en contact avec une surface latérale du capteur, dans lequel l'entretoise est configurée pour centrer le capteur à l'intérieur du boîtier du capteur par rapport à un axe longitudinal du boîtier du capteur, et dans lequel l'entretoise est configurée pour commander une profondeur du capteur à l'intérieur du boîtier du capteur le long d'un axe longitudinal du boîtier du capteur ou l'alignement du capteur par rapport à un axe longitudinal du boîtier du capteur.

14. Procédé selon la revendication 12 ou 13, dans lequel l'entretoise comprend un premier matériau polymère et le procédé comprenant en outre l'adhérence (1440) du capteur à l'entretoise par un deuxième matériau polymère positionné au moins partiellement dans l'évidement et l'adhérence de l'entretoise au boîtier du capteur par un troisième matériau polymère en contact avec la surface proximale de l'entretoise.

15. Procédé selon l'une quelconque des revendications 12 à 14, dans lequel en outre l'entretoise est produite par fabrication additive monocomposante ou multicomposante.
